(19) Europäisches Patentamt
European Patent Office
Office européen des brevets

(11) Veröffentlichungsnummer: **0 584 159 B1**

(12) **EUROPÄISCHE PATENTSCHRIFT**

(45) Veröffentlichungstag der Patentschrift: **27.12.95**

(51) Int. Cl.$^{6}$: **A23L 1/30**, A61K 31/22, C11C 3/02

(21) Anmeldenummer: **92910171.5**

(22) Anmeldetag: **13.05.92**

(86) Internationale Anmeldenummer: **PCT/EP92/01049**

(87) Internationale Veröffentlichungsnummer: **WO 92/20241 (26.11.92 92/29)**

(54) **NÄHRSTOFFZUBEREITUNG**

(30) Priorität: **16.05.91 DE 4116004**

(43) Veröffentlichungstag der Anmeldung: **02.03.94 Patentblatt 94/09**

(45) Bekanntmachung des Hinweises auf die Patenterteilung: **27.12.95 Patentblatt 95/52**

(84) Benannte Vertragsstaaten: **AT BE CH DE DK ES FR GB GR IT LI LU NL SE**

(56) Entgegenhaltungen:
**EP-A- 0 216 419**
**EP-A- 0 391 431**
**WO-A-91/03944**
**US-A- 3 579 548**
**EP-A- 0 366 480**
**WO-A-90/12080**
**FR-A- 1 600 887**

(73) Patentinhaber: **Fresenius AG**
**Gluckensteinweg 5**
**D-61350 Bad Homburg (DE)**

(72) Erfinder: **SOMMERMEYER, Klaus**
**Kapersburgstrasse 6B**
**D-6365 Rosbach v.d.H. (DE)**

(74) Vertreter: **Dr. Fuchs, Dr. Luderschmidt Dr. Mehler, Dipl.-Ing Weiss Patentanwälte**
**Postfach 46 60**
**D-65036 Wiesbaden (DE)**

EP 0 584 159 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Background der Erfindung

Die vorliegende Erfindung betrifft eine, ein Energiesubstrat aufweisende Nährstoffzubereitung für Menschen, die insbesondere für Urämiker und zur klinischen Ernährung geeignet ist.

Patienten, die klinisch ernährt werden müssen, haben häufig einen schlechten Ernährungszustand, wie er bei schwerem Trauma, wie zum Beispiel schweren und umfangreichen Verbrennungen, operativem Trauma, schweren Körperverletzungen, verschiedenen Formen von Krebs oder Sepsis, vorliegt. Bei solchen Zuständen ist das Stoffwechselbild kompliziert.

Im Stoffwechsel des Körpers wird aus Kohlenhydraten und Fetten eine Anzahl von Metaboliten synthetisiert, die der weiteren Oxidation unterliegen und dabei unterschiedliche Mengen an Energie produzieren. Beispiele für solche Metaboliten sind die Ketonkörper, die für den Körper wichtige alternative Energiesubstrate darstellen. Im Hungerzustand werden sie in großen Mengen gebildet und werden bei längerem Hungerzustand als Energiequelle wichtiger als Glucose. Die Möglichkeit der Verwertung von Ketonkörpern verhindert den schädlichen Abbau (Katabolie) von Muskelprotein für die Gluconeogenese, setzt damit den Verlust an wertvollem Körperprotein herab und übt so einen proteinsparenden Effekt aus.

In den peripheren Proteindepots (Muskeln) werden im wesentlichen die verzweigtkettigen Aminosäuren Leucin, Isoleucin und Valin, hauptsächlich Leucin, oxidiert, was bedeutet, daß bei diesen Patienten sich der Pool der verzweigtkettigen Aminosäuren graduell erschöpft, woraus wiederum eine verminderte Proteinsynthese in der Leber resultiert.

Ketonkörper rücken daher zunehmend in das Interesse als Energiesubstrat sowohl bei der enteralen als auch bei der parenteralen Ernährung über ihren bisherigen Einsatz bei Dialysepatienten bzw. bei Patienten, die eingeschränkte Nierenfunktion aufweisen, hinaus.

Es wurden bereits zahlreiche Versuche unternommen, eine Methode zu finden, durch die Ketonkörper und ähnliche Substanzen dem Körper wirksam zugeführt werden können. Von besonders hohem Interesse sind dabei die verzweigtkettigen Ketosäuren, insbesondere Ketoleucin, Ketoisoleucin und Ketovalin.

Die Möglichkeit des Einsatzes von Ketosäuren ist jedoch aus verschiedenen Gründen limitiert. In wäßriger Lösung sind sie praktisch nicht zu verarbeiten, da sie eine sehr geringe Stabilität aufweisen und darüber hinaus auch eine erheblich große Belastung mit sich bringen. Ferner müssen sie in Form von Salzen eingesetzt werden, was das Risiko in sich birgt, daß bei ihrer Verabreichung übermäßige Mengen an Kationen, zum Beispiel Natrium- und Kalium-Kationen, aufgenommen werden. Außerdem sind die Ketosäuren und deren Salze in Wasser löslich und erhöhen damit den osmotischen Druck des Nahrungsmittels, was die entsprechenden nachteiligen Effekte, wie zum Beispiel zu schnelle Passage durch den Darm und Diarrhoe, bewirkt.

Eine Umgehung dieser Problematik wird durch die etwas stabileren verzweigtkettigen $\alpha$-Hydroxysäuren erreicht, die zunächst allerdings allein als stickstofffreie Aminosäuresubstitute verabreicht wurden. Gemäß der PCT-Anmeldung WO 90/02547 werden $\alpha$-Hydroxysäuren und deren pharmazeutisch akzeptablen Metallsalze als Präcursoren der verzweigtkettigen Ketosäuren eingesetzt. Diese $\alpha$-Hydroxysäuren werden nach entsprechender Verstoffwechselung zu Ketosäuren als Energiesubstrat bei der enteralen und parenteralen Ernährung metabolisch genützt.

In der EP 0 367 734 werden Energiesubstrate für die klinische Ernährung eingesetzt, die mindestens einen Ester von Glycerin und mindestens eine verzweigtkettige Hydroxycarbonsäure enthalten. Als Beispiele für solche Hydroxycarbonsäuren werden L-$\alpha$-Hydroxy-isocapronsäure, $\alpha$-Hydroxy-$\beta$-methylvaleriansäure und L-$\alpha$-Hydroxy-isovaleriansäure genannt.

Die Verwendung von Triglyceriden mit geringeren Kalorien in Nahrungsmitteln wird in der PCT-Anmeldung WO 91/03944 vorgeschlagen, wobei mit der Nahrungsaufnahme gleichzeitig die Kalorienaufnahme verringert werden soll. Bei den zu diesem Zweck konzipierten Triglyceriden sind die Hydroxygruppen in Stellung 1 und 3 des Glycerins mit gesättigten langkettigen Fettsäuren (16 - 40 Kohlenstoffatome) verestert und ist die Hydroxygruppe in Stellung 2 des Glycerins mit einer kurzkettigen Säure (2 - 10 Kohlenstoffatome), die entweder gesättigt oder ungesättigt oder gerad- oder verzweigtkettig sein kann, verestert.

Die Verwendung von Glycerinestern in Nahrungsmittelgemischen zur Behandlung von Fettleibigkeit ist auch aus der US-PS 3,579,548 bekannt. Bei den dort eingesetzten Glycerinestern handelt es sich um Glycerinester von Seite 5 $\alpha$-verzweigtkettigen Carbonsäuren. Mit diesen Triglyceriden sollen wenig Kalorien bereitgestellt werden, d.h. diese Triglyceride sollen nicht in gleichem Maße wie übliche Triglyceride verdaut und absorbiert werden.

Gemäß der FR-PS 1.600.887 wird Glycerin-tri-isobutyrat als Bestandteil von Nahrungsmitteln, insbesondere von Nahrungsmitteln für Zuchttiere, vorgeschlagen.

Aus der WO-A- 90/12080 sind synthetische Triglyceride und deren Verwendung zur Behandlung von Patienten mit Intestinalproblemen bekannt. Die synthetischen Triglyceride besitzen die Formel

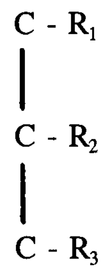

wobei $R_1$, $R_2$ und $R_3$ Fettsäurereste bedeuten, wobei mindestens einer, jedoch nicht alle der Reste $R_1$, $R_2$ und $R_3$ einen Rest einer kurzkettigen Fettsäure mit 2-5 C-Atomen im Kohlenstoffgerüst bedeuten, wobei, wenn $R_1$ und $R_3$ von der gleichen kurzkettigen Fettsäure abgeleitet sind, $R_2$ keine langkettige Fettsäure mit 14 bis 22 Kohlenstoffatomen im Kohlenstoffgerüst bedeutet, und jene Fettsäuren, die keine kurzkettigen Fettsäuren sind, langkettige (14 bis 24 Kohlenstoffatome) oder mittelkettige (6 bis 12 Kohlenstoffatome) Fettsäuren sind. Vorzugsweise bedeutet $R_2$ entweder eine kurzkettige Fettsäure ($C_2$ bis $C_5$) oder eine mittelkettige Fettsäure mit 6 bis 12 Kohlenstoffatomen. Insbesondere bedeutet $R_2$ eine mittelkettige Fettsäure und $R_1$ und $R_3$ kurzkettige Fettsäuren, vorzugsweise mit 3 bis 5 Kohlenstoffatomen.

In der EP-A- 0 391 431 werden Diacylglycerine sowie deren Verwendung für kosmetische Zwecke beschrieben. Diese Diacylglycerine besitzen einen von einer spezifischen verzweigtkettigen gesättigten Fettsäure abgeleiteten Rest und einen von einer spezifischen geradkettigen gesättigten Fettssäure abgeleiteten Rest, wobei der von einer geradkettigen gesättigten Fettsäure abgeleitete Rest 11 bis 17 Kohlenstoffatome und der von einer verzweigtkettigen gesättigten Fettsäure abgeleitete Rest 10 bis 18 Kohlenstoffatome aufweist, wobei einige Reste nicht enthalten sein können.

Die EP-A- 0 216 419 beschreibt ein Nahrungsfett, das für enterale und parenterale Produkte geeignet ist. Dieses Fett besteht im wesentlichen aus etwa 50 bis 100 Gew.-% Triglyceriden der Formel

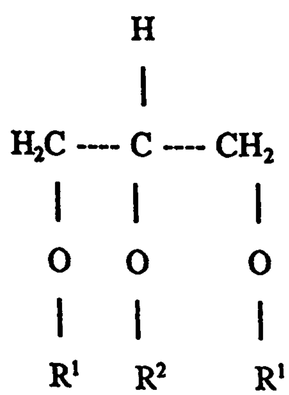

wobei jeder $R^1$ einen linearen $C_7$- bis $C_{11}$-gesättigten Acylrest, wie den n-Heptanoylrest, n-Octanoylrest, n-Nonanoylrest, n-Decanoylrest oder n-Undecanoylrest, bedeutet und der Rest $R^2$ von 0 bis etwa 90 % gesättigte Acylreste, wie n-Hepanoylrest, n-Octanoylrest, n-Nonanoylrest, n-Decanoylrest, n-Undecanoylrest, Lauroylrest, Myristoylrest, Palmittoylrest, Stearoylrest oder Gemische derselben, von 0 bis etwa 90 % Oleoylreste, von etwa 10 bis etwa 100 % Linoleoylreste und von 0 bis etwa 10 % Linolenoylreste umfaßt.

Aus der EP-A- 0 366 480 sind Zusammensetzungen für die Behandlung von Alzheimer-Erkrankung, verwandten Dementien und Epilepsie bekannt. Diese Zusammensetzungen bestehen aus a) etwa 13,0 bis 27,5 Gew.-%, vorzugsweise etwa 15,0 bis etwa 24,5 Gew.-%, insbesondere von etwa 16,7 bis etwa 22,2 Gew.-% mindestens einer Verbindung, wie Linolensäure oder Derivaten derselben, wobei die Derivate von Linolensäure sowohl physiologisch hydrolysierbar und pharmakologisch akzeptabel sind, und b) einer Balance zur Erzielung von 100 Gew.-% mindestens einer Verbindung, wie Linolensäure oder Derivaten derselben, wobei diese Derivate sowohl physiologisch hydrolysierbar als auch pharmakologisch akzeptabel sind.

Beschreibung der Erfindung

Erfindungsgemäß wurde überraschend gefunden, daß in Wasser schwer lösliche bzw. praktisch unlösliche Glyceride der allgemeinen Formel I

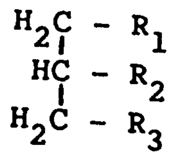

wobei einer der Reste $R_1$, $R_2$ oder $R_3$ einen Hydroxyrest oder einen Rest einer gesättigten oder ungesättigten Fettsäure mit 2 bis 24 Kohlenstoffatomen bedeuten kann und zwei oder drei der Reste $R_1$, $R_2$ oder $R_3$ einen Rest der allgemeinen Formel II

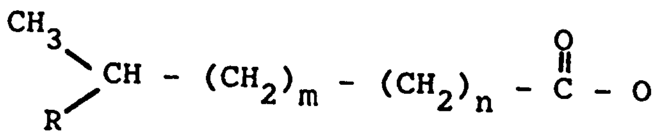

wobei R einen Methylrest oder einen Ethylrest, m 0 oder 1 und n 0 oder eine gerade Zahl von 2 bis 18 bedeuten, ausgenommen Glyceryl-triisobutyrat, für Menschen ausgezeichnete Energiesubstrate darstellen, insbesondere für Urämiker, und zur klinischen Ernährung geeignet sind und die vorstehend genannten Nachteile nicht aufweisen.

Diese Glyceride stellen stabile und leicht zu handhabende Verbindungen dar. Sie sind in Wasser schwer löslich bzw. praktisch nicht löslich und üben daher auch keinen osmotischen Druck aus. Sie werden im Körper erst im Verlauf ihrer Verstoffwechselung, z.B. über die $\alpha$- und/oder $\beta$-Oxidation zu den entsprechenden hydroxyanalogen Fettsäuren, insbesondere den Hydroxyanalogen der verzweigtkettigen Aminosäuren, umgewandelt, aus denen nach entsprechender Verstoffwechselung die entsprechenden verzweigtkettigen Ketosäuren gebildet werden, die als Energiesubstrat metabolisch genützt werden. Sie können ebenfalls unter Abbau des bei Urämikern vergrößerten Harnstoffpools enzymatisch zu den entsprechenden verzweigtkettigen L-Aminosäuren transaminiert werden. Sie ermöglichen die Reutilisation von stickstoffhaltigen Abbauprodukten, Proteinanabolismus bei gleichzeitigem Sinken des Serumharnstoffs sowie einer Verbesserung der Stickstoffbilanz und damit einer Verbesserung der urämischen Symptomatik, wodurch gegebenenfalls eine Dialyse hinausgeschoben werden kann.
Mit den erfindungsgemäßen Zubereitungen, die diese Glyceride enthalten, wird somit ein proteinsparender Effekt erzielt.

Weitere Vorteile der erfindungsgemäß angewandten Glyceride sind in der Kinetik der Verstoffwechselung zu sehen, da die Ketokörper nicht so exzessiv ansteigen können wie unter der Therapie mit den hydroxyanalogen Verbindungen. Die Verstoffwechselung der eingesetzten mittelkettigen Glyceride erfolgt zudem Carnitin- unabhängig.

Die Herstellung der erfindungsgemäß eingesetzten Glyceride erfolgt in an sich bekannter Weise wie sie beispielweise in "Analyse der Fette und Fettprodukte einschließlich der Wachse, Harze und verwandten Stoffe", H.P. Kaufmann, Springer Verlag, 1958 beschrieben ist. Ferner sind die erfindungsgemäß eingesetzten Glyceride unter Anwendung der bekannten Technologien der Herstellung von Fettemulsionen leicht zu verarbeiten und bieten als wesentlichen Vorteil keinerlei Stabilitätsprobleme.

Detallierte Beschreibung der Erfindung

Die in den erfindungsgemäßen Zubereitungen eingesetzten Glyceride besitzen die allgemeine Formel I

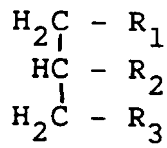

worin einer der Reste $R_1$, $R_2$ oder $R_3$ einen Hydroxyrest oder einen Rest einer gesättigten oder ungesättigten Fettsäure mit 2 bis 24 Kohlenstoffatomen bedeuten kann und zwei oder drei der Reste $R_1$, $R_2$ oder $R_3$ einen Rest der allgemeinen Formel II

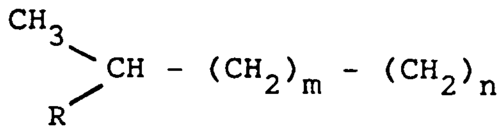 II

worin R einen Methylrest oder einen Ethylrest, m 0 oder 1 und n 0 oder eine gerade Zahl von 2 bis 18 bedeuten, ausgenommen Glyceryl-triisobutyrat, bedeuten.

Vorzugsweise bedeutet in der allgemeinen Formel II m 1 und n 0 oder eine gerade Zahl von 2 - 6, insbesondere 0, 2 oder 4.

Beispiele für geeignete Reste der Formel II sind die Reste der Formeln III bis V

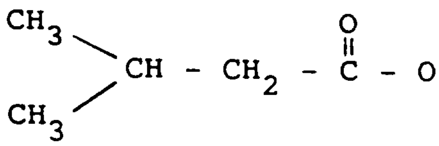 III

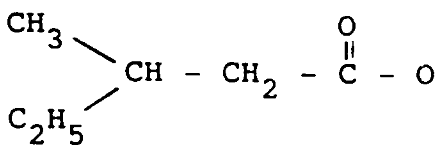 IV

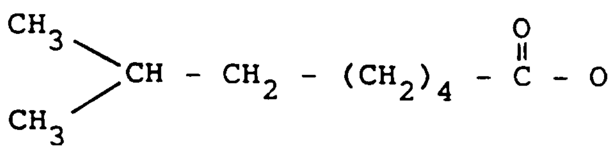 V

aus denen im Körper nach entsprechender Verstoffwechselung, z.B. nach entsprechender $\alpha$ und/oder $\beta$-Oxidation, die entsprechenden $\alpha$ Hydroxysäuren gebildet werden, die in die entsprechenden $\alpha$-Ketoanalogen, d.h. Ketovalin, Ketoisoleucin bzw. Ketoleucin umgewandelt werden.

Sind die Reste $R_1$, $R_2$ und/oder $R_3$ der Formel I von Anteisofettsäuren abgeleitet, so sind sie bevorzugt von der L-Form dieser Säuren abgeleitet.

Vorzugsweise bedeuten in der allgemeinen Formel I alle drei Reste $R_1$, $R_2$ und $R_3$ einen Rest der allgemeinen Formel II, wobei die Reste $R_1$, $R_2$ und $R_3$ den gleichen Rest der allgemeinen Formel II oder verschiedene Reste der allgemeinen Formel II bedeuten können. Vorzugsweise bedeuten alle Reste $R_1$, $R_2$

oder $R_3$ den gleichen Rest der allgemeinen Formel II.

Spezielle Beispiele für Glyceride, die in den erfindungsgemäßen Zubereitungen Verwendung finden, sind

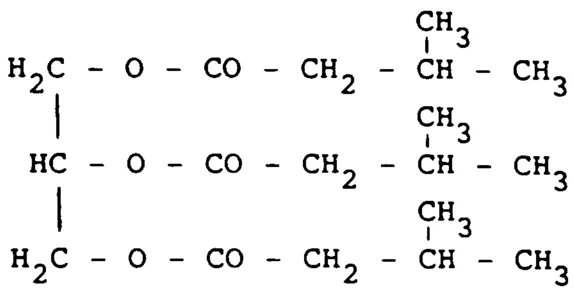

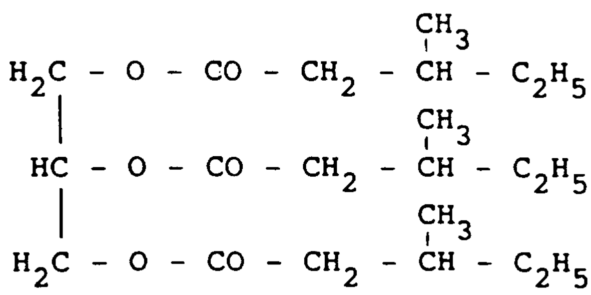

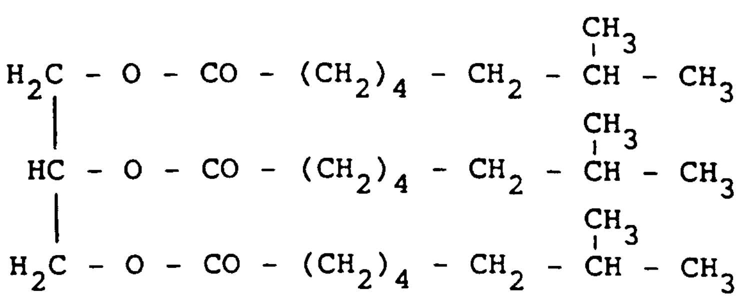

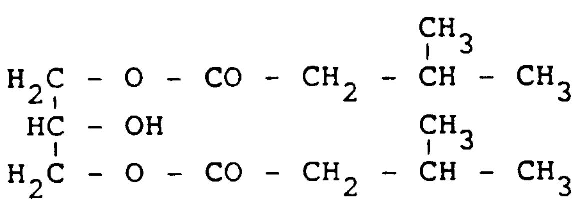

Es können auch Gemische der Glyceride untereinander eingesetzt werden.

Die in den erfindungsgemäßen Zubereitungen enthaltenen Glyceride können auch in Form von tierischen Fetten und/oder Ölen vorliegen bzw. eingesetzt werden. Beispielsweise können sie in Form von Fischöl und/oder Wiederkäuerfett oder in Form von Fraktionen dieser Fette und/oder Öle vorliegen, in denen die die verzweigten Fettsäuren enthaltenden Triglyceride angereichert sind.

Die erfindungsgemäßen Zubereitungen enthalten die Glyceride in einer Menge von 0,2 bis 100% (Gewicht/Volumen), vorzugsweise in einer Menge von 0,2 bis 35 % (Gewicht/Volumen).

Die erfindungsgemäßen Zubereitungen können sowohl enteral als auch parenteral verabreicht werden. Für die Verabreichungen sind die üblichen Verabreichungsformen, zum Beispiel Emulsionen oder Ölkapseln, geeignet.

Die erfindungsgemäß verwendeten Glyceride sind ölige Substanzen. Sie können allein oder zusammen mit bekannten üblichen pharmazeutisch verträglichen Substanzen verwendet werden. Da sie in Wasser schwer bzw. nicht löslich sind, müssen sie, insbesondere zur intravenösen Infusion, in eine Emulsion überführt werden. Die Glyceride sind nur soweit sie ausreichende Unlöslichkeit in Wasser aufweisen, dafür geeignet, in Emulsionsform gebracht zu werden. Die Herstellung der Emulsion erfolgt in an sich bekannter Weise nach bekannten Techniken, beispielsweise wie in der DE-PS 37 22 540 beschrieben. Die erfindungsgemäßen Emulsionen sind stabil und weisen Zusammensetzungen und Eigenschaften auf, die es gestatten, sie an Mensch und Tier sowohl enteral als auch parenteral zu verabreichen. Zur Herstellung der Emulsion wird geeigneterweise eine sterile, pyrogenfreie Wasserphase verwendet. Geeignete Emulgatoren sind Phospholipide, pflanzlichen oder tierischen Ursprungs bzw. Fraktionen dieser Substanzen. Vorzugsweise wird fraktioniertes Eilecithin eingesetzt. Als Hilfsstoffe zur Isotonisierung der äußeren wässrigen Phase kommen Glycerin oder andere pharmazeutisch akzeptable Polyole, wie z.B. Sorbit, zum Einsatz.

Geeignete Coemulgatoren sind Natrium- oder Kaliumsalze längerkettiger Fettsäuren, z.B. Natrium- oder Kaliumsalze von Ölsäure, Palmitinsäure oder Stearinsäure.

Die Emulsionen können ebenfalls Aminosäuren, Fette synthetischen, pflanzlichen oder tierischen Ursprungs, Kohlenhydrate, Vitamine und/oder Spurenelemente enthalten. Beispiele für geeignete Fette sind Sojaöl, Safloröl, Olivenöl, Baumwollsamenöl, Sonnenblumenöl und Triglyceride mit unverzweigter mittlerer Kohlenstoffkette (MCT) und/oder andere physiologisch akzeptable Triglyceride pflanzlichen, tierischen oder synthetischen Ursprungs. Bevorzugt ist dabei ein Zusatz von hochfraktionierten Fischölen, da diese in geringen Mengen geeignete, verzweigtkettige Triglyceride enthalten und somit als natürliche Quellen für verzweigtkettige Triglyceride ausgenützt werden. Weiterhin können Fettfraktionen von Depotfetten von Wiederkäuern ebenfalls in diesem Sinne zum Tragen kommen. Der Einsatz von Triglyceridfraktionen aus diesen beiden bevorzugten natürlichen tierischen Quellen hat darüber hinaus den Vorteil, daß dabei die L-Form der verzweigtkettigen Anteisofettsäuren vorhanden ist, wie sie z.B. auch im asymetrischen C-Atom der Alkylkette des Isoleucins vorliegt. Diese Fette können in den Emulsionen in einer Menge von 2 bis 30 % enthalten sein.

Die erfindungsgemäßen Zubereitungen können ebenfalls 0,5 bis 10 % acetylierte Mono- oder Diglyceride von gesättigten oder ungesättigten Fettsäuren mit 6 bis 24 Kohlenstoffatomen enthalten.

Emulsionen stellen die bevorzugte Form der erfindungsgemäßen Zubereitungen dar und können parenteral oder enteral verabreicht werden. Die Glyceride bzw. die erfindungsgemäßen Zubereitungen könnne jedoch auch als Zusätze zur normalen Diät oder in irgendeiner anderen üblichen Zubereitungsform verwendet werden. Eine weitere geeignete Zubereitungsform sind Ölkapseln, wobei die Kapseln aus Gelatine, z.B. Weichgelatine oder einem anderen geeigneten Material, vorzugsweise aus Weichgelatine bestehen.

Die in den erfindungsgemäßen Zubereitungen eingesetztn Glyceride sind gut verträglich und bei den üblicherweise anwendbaren Dosen nicht toxisch.

Die erfindungsgemäßen Zubereitungen finden bei Mensch und Tier, die sich in katabolem Zustand befinden, Anwendung. Sie sind geeignet zur Behandlung von schwerem Trauma und sepsis und insbesondere zur Behandlung von Urämikern, zum Beispiel bei chronischer Niereninsuffizienz, Störungen der Harnstoffsynthese, zur Ernährungstherapie bei akutem Nierenversagen.

Die nachfolgenden Beispiele dienen der Erläuterung der vorliegenden Erfindung.

<u>Beispiel 1</u>

Herstellung von Glyceryl-triisovalerinat

In einem mit Rührer und Rückflußkühler mit Wasserabscheider ausgestatteten Reaktionsgefäß wurden unter Rühren 1 Mol Glycerin und 5 ml konzentrierte Schwefelsäure mit 3,5 Mol Isovaleriansäure versetzt und unter Rückfluß erhitzt, wobei zur Entfernung des Wassers 700 ml Benzol zugesetzt wurden. Das erhaltene Reaktionsprodukt wurde in üblicher Weise gereinigt. Es wurde mittels Gaschromatographie auf seine Identität und Reinheit geprüft und entsprach dabei den Literaturangaben bp = 330 -$5^{763}$, d = $0{,}9984_4^{20}$ , nD = 1,435.

Beispiel 2

Herstellung von Glyceryl-tri(3-methylvalerinat)

Das Verfahren von Beispiel 1 wurde wiederholt mit der Ausnahme, daß anstelle von Isovaleriansäure 3-Methylvaleriansäure verwendet wurde.

Beispiel 3

Herstellung von Glyceryl-tri(4-methylvalerinat)

Das Verfahren von Beispiel 1 wurde wiederholt mit der Ausnahme, daß anstelle von Isovaleriansäure 4-Methylvaleriansäure verwendet wurde.

Beispiel 4

Herstellung von Ölkapseln

Die gemäß Beispiel 1 hergestellte Verbindung wurde in Weichgelatinekapseln gebracht, wobei pro Kapsel 0,5 g der Verbindung verwendet wurde.

Beispiel 5

Herstellung einer Emulsion der folgenden Zusammensetzung:

100 g hochfraktioniertes Fischöl
100 g Glyceryl-triisovalerinat
12 g Eilecithin, fraktioniert
0,3 g Natriumoleat
25 g Glycerin (wasserfrei)
ad 1000 ml Aqua ad injektabilia

Unter Verwendung der vorstehend genannten Komponenten wurde eine Emulsion hergestellt, wobei dafür gesorgt wurde, daß die Komponenten und Mischungen ständig unter Stickstoffatmosphäre gehalten wurden. 12 g Eilecitin wurden in ca. 2 Minuten unter ständigem Rühren zu 75 ml destilliertem Wasser für Injektionszwecke, das auf 55 bis 60 °C temperiert war, zugesetzt. Die erhaltene Mischung wurde noch 15 Minuten weitergerührt. Parallel wurden zu 25 ml destilliertem Wasser für Injektionszwecke , das ebenfalls auf 55 bis 60 °C temperiert war, 25 g Glycerin (100 %ig) und 0,3 g Natriumoleat zugesetzt und langsam unter Rühren gelöst. Die erhaltene Lösung wurde weiterhin bei 55 bis 60 °C gehalten und unter Stickstoffdruck innerhalb von 10 Minuten durch ein 0,2 µm Membranfilter der vorbereiteten Wasser/Lecithin-Mischung zugesetzt. 100 g Triglycerid wurden auf 55 bis 60 °C erwärmt und durch ein Nylon- Membranfilter mit einer Porengröße von 0,2 µm innerhalb von 20 bis 25 Minuten direkt der vorbereiteten wäßrigen Mischung aus Lecithin, Glycerin und Natriumoleat unter ständigem Rühren, beispielsweise unter Verwendung eines mechanischen Hochfrequenzgerätes (Ultra-Turrax) zum Emulgieren zusammen mit einem Rührwerk, zugesetzt. Nach beendeter Zugabe des Glycerids wurde die gebildete Rohemulsion 25 Minuten weiteremulgiert. Während der Herstellung der Rohemulsion wurde diese auf einer Temperatur im Bereich von 60 bis 65 °C gehalten und ständig mit Stickstoff überlagert.

Nach Abschalten des mechanischen Hochfrequenzgerätes wurde die Rohemulsion unter leichtem Rühren durch ein Membranfilter mit einer durchschnittlichen Porengröße von 40 µm unter einem Stickstoffdruck von ca. 0,5 bar in einem für die Herstellung von Fettemulsionen geeigneten zweistufigen Homogenisator (erste Stufe 400 bar, zweite Stufe 100 bar) gebracht und homogenisiert. Die Temperatur betrug nach dem ersten Homogenisierschritt etwa 70 °C und nach dem zweiten Homogenisierschritt etwa 80 °C. Die Emulsion wurde anschließend auf 70 °C abgekühlt und in einen Lagertank gebracht, der mit Stickstoff überschichtet war. Man ließ die Emulsion unter gelegentlichem langsamen Umrühren ruhen. Danach wurde die Emulsion noch zwei weiteren Homogenisierschritten unterzogen, wobei die Temperatur nach dem dritten Homogenisierschritt bei 75 °C und nach dem vierten Homogenisierschritt bei 80 bis 85 °C lag. Anschließend wurde die Emulsion so stark wie möglich abgekühlt und in eine Vorlage gebracht, die 757 ml auf 12 °C gekühltes destilliertes Wasser enthielt. Dieser Vorgang wurde ebenfalls unter Stickstoffatmosphäre durchgeführt. Unter gelegentlichem langsamen Rühren wurde die Emulsion weiter auf 8 bis 9 °C gekühlt. Nach Erreichen dieser Temperatur wurde der Rührer abgeschaltet.

Der pH-Wert der Emulsion wurde geprüft, Abweichungen vom vorgegebenen Sollwert (pH-Wert von 8,7 bis 8,8) wurden durch Zusatz einer entsprechenden Menge I n Natronlauge korrigiert. Die erhaltene Fettemulsion wurde in einem Kühltank unter Stickstoffatmosphäre aufbewahrt und vor dem Abfüllen durch ein Membranfilter mit einer Porengröße von 2 bis 8 µm filtriert. Der Abfülldruck betrug maximal 0,5 bar. Die Abfüllung erfolgte in Glasbehältnisse, die anschließend verschlossen wurden. Die Fettemulsionen müssen vor Licht und Sauerstoff geschützt werden. Die Abfüllung wurde unter Stickstoff vorgenommen, damit der Sauerstoffgehalt in den Behältnissen möglichst gering gehalten wurde.

Beispiel 6

Unter Verwendung von Glyceryl-triisovalerinat wurde eine Nährstoffzubereitung hergestellt, in der das Glycerid in einer Menge von 5 bis 15 % enthalten war. Die Zusammensetzung der Nährstoffzubereitung war folgende:

| | |
|---|---|
| Milch und Sojaprotein | 3,8 g |
| Glyceryl-triisovalerinat | 5 g |
| essentielle Fettsäuren | 1,6 g |
| Glucose | 0,1 g |
| Maltose | 0,4 g |
| Rohrzucker | 3,0 g |
| Polysaccharide | 10,2 g |
| Na | 3,3 mMol |
| K | 3,2 mMol |
| Cl | <3,3 mMol |
| Ca | 50 mg |
| Mg | 13 mg |
| P | 60 mg |
| Fe | 1,0 mg |
| Zn | 0,75 mg |
| Cu | 0,15 mg |
| Jod | 7,5 µg |
| Wasser aufgefüllt auf 100 ml. | |

Diese Nährstoffzubereitung kann noch mit dem täglichen Bedarf an wasserlöslichen und fettlöslichen Vitaminen ergänzt werden.

**Patentansprüche**

**Patentansprüche für folgende Vertragsstaaten: AT, BE, CH, LI, DE, DK, FR, GB, IT, LU, NL, SE**

**1.** Nährstoffzubereitung für Menschen, dadurch gekennzeichnet, daß sie mindestens ein in Wasser schwer lösliches bzw. praktisch nicht lösliches Glycerid der allgemeinen Formel I

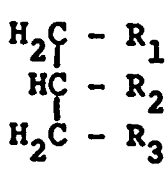

I

wobei einer der Reste $R_1$, $R_2$ oder $R_3$ einen Hydroxyrest oder einen Rest einer gesättigten oder ungesättigten Fettsäure mit 2 bis 24 Kohlenstoffatomen bedeuten kam und zwei oder drei der Reste $R_1$, $R_2$ oder $R_3$ einen Rest der allgemeinen Formel II

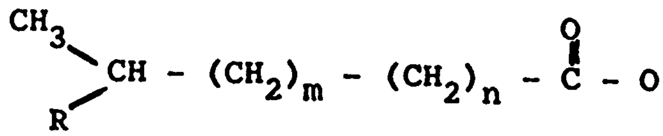 II

wobei R einen Methylrest oder einen Ethylrest, m 0 oder 1 und n 0 oder eine gerade Zahl von 2 bis 18 bedeuten, ausgenommen Glyceryl-triisobutyrat, enthält.

**2.** Zubereitung nach Patentanspruch 1, dadurch gekennzeichnet, daß m 1 und n 0 oder eine gerade Zahl von 2 bis 6 bedeuten.

**3.** Zubereitung nach Patentanspruch 2, dadurch gekennzeichnet, daß n 0, 2 oder 4 bedeutet.

**4.** Zubereitung nach Patentansprüchen 1 bis 3, dadurch gekennzeichnet, daß alle Reste $R_1$, $R_2$ und $R_3$ der allgemeinen Formel I jeweils den Rest der Formel III

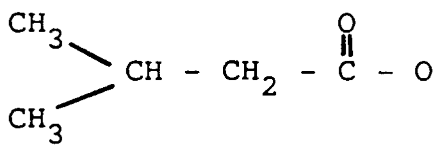 III

bedeuten.

**5.** Zubereitung nach Patentansprüchen 1 bis 3, dadurch gekennzeichnet, daß alle Reste $R_1$, $R_2$ und $R_3$ der allgemeinen Formel I jeweils den Rest der Formel IV

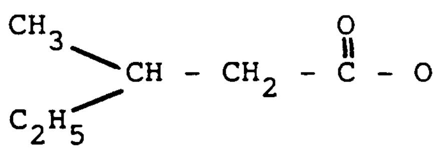 IV

bedeuten.

**6.** Zubereitung nach Patentanspruch 1 bis 5, dadurch gekennzeichnet, daß sie das Glycerid in einer Menge von 0,2 - 100 % (Gewicht/Volumen) enthält.

**7.** Zubereitung nach Patentanspruch 6, dadurch gekennzeichnet, daß sie das Glycerid in einer Menge von 0,2 - 35 % (Gewicht/Volumen) enthält.

**8.** Zubereitung nach Patentanspruch 1 bis 7, dadurch gekennzeichnet, daß sie ein Gemisch der Glyceride der Formel I enthält.

**9.** Zubereitung nach einem der vorstehenden Patentansprüche, dadurch gekennzeichnet, daß die Glyceride in Form von tierischen Fetten und/oder Ölen vorliegen.

**10.** Zubereitung nach Patentanspruch 9, dadurch gekennzeichnet, daß sie in Form von Fischöl und/oder Wiederkäuerfett vorliegen.

**11.** Zubereitung nach Patentanspruch 9 oder 10, dadurch gekennzeichnet, daß sie in Form von an verzweigte Fettsäuren enthaltenden Triglyceriden angereicherten Fraktionen dieser Fette und/Öle vorliegen.

**12.** Zubereitung nach Patentanspruch 1 bis 11, dadurch gekennzeichnet, daß sie in Form einer Emulsion des Glycerids in wäßriger Phase vorliegt.

**13.** Zubereitung nach Patentanspruch 12, dadurch gekennzeichnet, daß sie ebenfalls 2 - 30 % fraktioniertes Sojaöl, Safloröl, Olivenöl, Fischöl, Wiederkäuerfett, Baumwollsamenöl, Sonnenblumenöl, Triglyceride mit einer unverzweigten Kohlenstoffkette mittlerer Länge (MCT) und/oder andere physiologisch akzeptable Triglyceride pflanzlichen, tierischen oder synthetischen Ursprungs enthält.

**14.** Zubereitung nach einem der Ansprüche 12 oder 13, dadurch gekennzeichnet, daß sie ebenfalls 0,5 - 10 % acetylierte Mono- oder Diglyceride von gesättigten oder ungesättigten Fettsäuren mit 6 bis 24 Kohlenstoffatomen enthält.

**15.** Verwendung von mindestens einem in Wasser schwer löslichem bzw. praktisch nicht löslichem Glycerid der allgemeinen Formel I

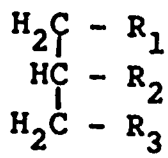

I

wobei einer der Reste $R_1$, $R_2$ oder $R_3$ einen Hydroxyrest oder einen Rest einer gesättigten oder ungesättigten Fettsäure mit 2 bis 24 Kohlenstoffatomen bedeuten kann und zwei oder drei der Reste $R_1$, $R_2$ oder $R_3$ einen Rest der allgemeinen Formel II

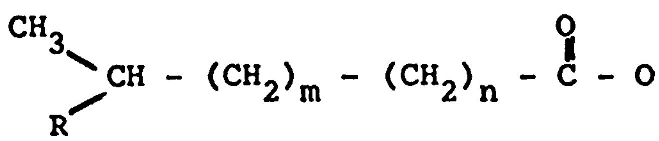

II

wobei R einen Methylrest oder einen Ethylrest, m 0 oder 1 und n 0 oder eine gerade Zahl von 2 bis 18 bedeuten, zur Herstellung einer Nährstoffzubereitung für Menschen, die sich im katabolen Zustand befinden.

**Patentansprüche für folgende Vertragsstaaten : ES, GR**

**1.** Verwendung von mindestens einem in Wasser schwer löslichem bzw. praktisch nicht löslichem Glycerid der allgemeinen Formel I

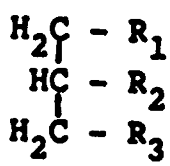

I

wobei einer der Reste $R_1$, $R_2$ oder $R_3$ einen Hydroxyrest oder einen Rest einer gesättigten oder ungesättigten Fettsäure mit 2 bis 24 Kohlenstoffatomen bedeuten kann und zwei oder drei der Reste $R_1$, $R_2$ oder $R_3$ einen Rest der allgemeinen Formel II

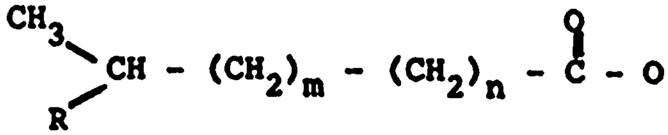

II

wobei R einen Methylrest oder einen Ethylrest, m 0 oder 1 und n 0 oder eine gerade Zahl von 2 bis 18 bedeuten, ausgenommen Glyceryl-triisobutyrat, zur Herstellung einer Nährstoffzubereitung für Menschen.

2. Verwendung nach Patentanspruch 1, dadurch gekennzeichnet, daß m 1 und n 0 oder eine gerade Zahl von 2 bis 6 bedeuten.

3. Verwendung nach Patentanspruch 2, dadurch gekennzeichnet, daß n 0, 2 oder 4 bedeutet.

4. Verwendung nach Patentansprüchen 1 bis 3, dadurch gekennzeichnet, daß alle Reste $R_1$, $R_2$ und $R_3$ der allgemeinen Formel I jeweils den Rest der Formel III

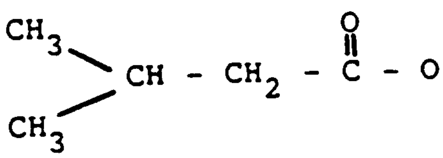

III

bedeuten.

5. Verwendung nach Patentansprüchen 1 bis 3, dadurch gekennzeichnet, daß alle Reste $R_1$, $R_2$ und $R_3$ der allgemeinen Formel I jeweils den Rest der Formel IV

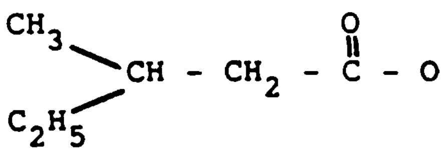

IV

bedeuten.

6. Verwendung nach Patentanspruch 1 bis 5, dadurch gekennzeichnet, daß das Glycerid in der Zubereitung Menge von 0,2 - 100 % (Gewicht/Volumen) enthalten ist.

7. Verwendung nach Patentanspuch 6, dadurch gekennzeichnet, daß das Glycerid in der Zubereitung in einer Menge von 0,2 - 35 % (Gewicht/Volumen) enthalten ist.

8. Verwendung nach Patentanspruch 1 bis 7, dadurch gekennzeichnet, daß man ein Gemisch der Glyceride der Formel I verwendet.

9. Verwendung nach einem der vorstehenden Patentansprüche, dadurch gekennzeichnet, daß man die Glyceride in Form von tierischen Fetten und/oder Ölen verwendet.

10. Verwendung nach Patentanspruch 9, dadurch gekennzeichnet, daß man die Glyceride in Form von Fischöl und/oder Wiederkäuerfett verwendet.

**11.** Verwendung nach Patentanspruch 9 oder 10, dadurch gekennzeichnet, daß man die Glyceride in Form von an verzweigte Fettsäuren enthaltenden Triglyceriden angereicherten Fraktionen dieser Fette und Öle verwendet.

**12.** Verwendung nach Patentanspruch 1 bis 11, dadurch gekennzeichnet, daß man die Zubereitung in Form einer Emulsion des Glycerids in wässriger Phase herstellt.

**13.** Verwendung nach Patentanspruch 12, dadurch gekennzeichnet, daß man ebenfalls 2 - 30 % fraktioniertes Sojaöl, Safloröl, Olivenöl, Fischöl, Wiederkäuerfett, Baumwollsamenöl, Sonnenblumenöl, Triglyceride mit einer unverzweigten Kohlenstoffkette mittlerer Länge (MCT) und/oder andere physiologisch akzeptable Triglyceride pflanzlichen, tierischen oder synthetischen Ursprungs verwendet.

**14.** Verwendung nach einem der Patentansprüche 12 oder 13, dadurch gekennzeichnet, daß man ebenfalls 0,5 - 10 % acetylierte Mono- oder Diglyceride von gesättigten oder ungesättigten Fettsäuren mit 6 bis 24 Kohlenstoffatomen verwendet.

**Claims**
**Claims for the following Contracting States : AT, BE, CH, LI, DE, DK, FR, GB, IT, LU, NL, SE**

**1.** Nutriment preparation for humans, characterized in that it contains at least one glyceride hard to dissolve respectively insoluble in water of the general Formula I

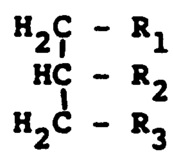

I

whereby one of the residues $R_1$, $R_2$ or $R_3$ may designate a hydroxy residue or a residue of a saturated or unsaturated fatty acid with 2 to 24 carbon atoms and two or three of the residues $R_1$, $R_2$ or $R_3$ may designate a residue of the general Formula II

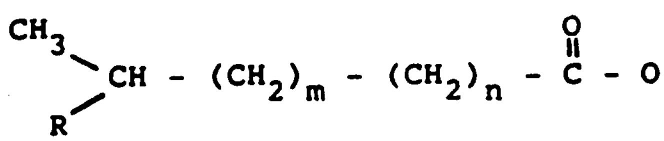

II

whereby R designates a methyl residue or an ethyl residue, m designates 0 or 1 and n designates 0 or an even number from 2 to 18, except for glyceril-triisobutyrate.

**2.** Preparation according to claim 1, characterized in that m designates 1 and n designates 0 or an even number from 2 to 6.

**3.** Preparation according to claim 2, characterized in that n designates 0, 2 or 4.

**4.** Preparation according to claims 1 to 3, characterized in that all residues $R_1$, $R_2$ and $R_3$ of the general Formula I respectively designate the residue of the Formula III

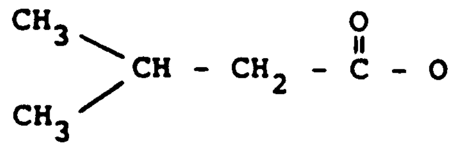

III

5. Preparation according to claims 1 to 3, characterized in that all residues $R_1$, $R_2$ and $R_3$ of the general Formula I respectively designate the residue of the Formula IV

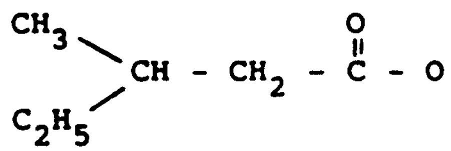

IV

6. Preparation according to claims 1 to 5, characterized in that it contains the glyceride in a quantity of 0.2 - 100% (weight/volume).

7. Preparation according to claim 6, characterized in that it contains the glyceride in a quantity of 0.2 - 35% (weight/volume).

8. Preparation according to claims 1 to 7, characterized in that it contains a mixture of the glycerides of Formula I.

9. Preparation according to one of the above claims, characterized in that the glycerides are present in the form of animal fats and/or oils.

10. Preparation according to claim 9, characterized in that they are present in the form of fish oil and/or ruminant fat.

11. Preparation according to claim 9 or 10, characterized in that they are present in the form of fractions of said fats and/or oils enriched by triglycerides containing branched fatty acids.

12. Preparation according to claims 1 to 11, characterized in that it is present in the form of an emulsion of the glyceride in aqueous phase.

13. Preparation according to claim 12, characterized in that it also contains 2 - 30 % fractionated soy oil, safflower oil, olive oil, fish oil, ruminant fat, cottonseed oil, sunflower oil, triglyceride with an unbranched carbon chain of medium length (MCT) and/or other physiologically acceptable triglycerides of vegetable, animal or synthetic origin.

14. Preparation according to one of claims 12 or 13, characterized in that it also contains 0.5 - 10% acetylized mono- or diglycerides of saturated or unsaturated fatty acids with 6 to 24 carbon atoms.

**15.** Use of at least one glyceride hard to dissolve respectively insoluble in water of the general Formula I

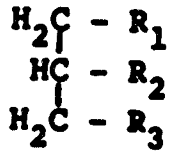

I

whereby one of the residues $R_1$, $R_2$ or $R_3$ may designate a hydroxy residue or a residue of a saturated or unsaturated fatty acid with 2 to 24 carbon atoms and two or three of the residues $R_1$, $R_2$ or $R_3$ may designate a residue of the general Formula II

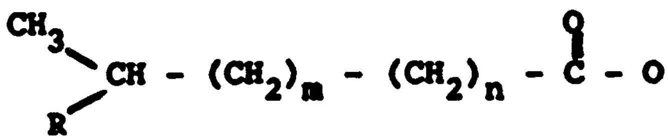

II

whereby R designates a methyl residue or an ethyl residue, m designates 0 or 1 and n designates 0 or an even number from 2 to 18, for nutriment preparation for humans being in catabolic condition.

**Claims for the following Contracting States : ES, GR**

**1.** Use of at least one glyceride hard to dissolve respectively insoluble in water of the general Formula I

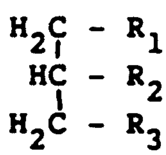

I

whereby one of the residues $R_1$, $R_2$ or $R_3$ may designate a hydroxy residue or a residue of a saturated or unsaturated fatty acid with 2 to 24 carbon atoms and two or three of the residues $R_1$, $R_2$ or $R_3$ may designate a residue of the general Formula II

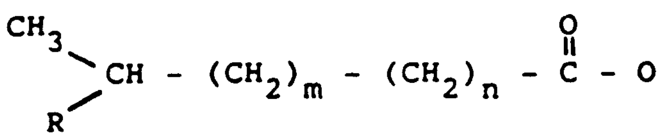

II

whereby R designates a methyl residue or an ethyl residue, m designates 0 or 1 and n designates 0 or an even number from 2 to 18, except for glyceril-triisobutyrate, for the nutriment preparation for humans.

**2.** Use according to claim 1, characterized in that m designates 1 and n designates 0 or an even number from 2 to 6.

**3.** Use according to claim 2, characterized in that n designates 0, 2 or 4.

4. Use according to claims 1 to 3, characterized in that all residues $R_1$, $R_2$ and $R_3$ of the general Formula I respectively designate the residue of the Formula III

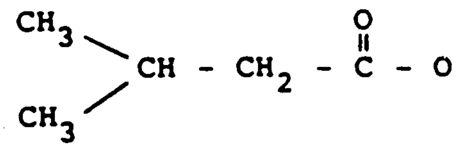

III

5. Use according to claims 1 to 3, characterized in that all residues $R_1$, $R_2$ and $R_3$ of the general Formula I respectively designate the residue of the Formula IV

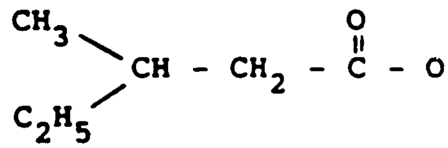

IV

6. Use according to claims 1 to 5, characterized in that it contains the glyceride in a quantity of 0.2 - 100% (weight/volume).

7. Use according to claim 6, characterized in that it contains the glyceride in a quantity of 0.2 - 35% (weight/volume).

8. Use according to claims 1 to 7, characterized in that it contains a mixture of the glycerides of Formula I.

9. Use according to one of the above claims, characterized in that the glycerides are present in the form of animal fats and/or oils.

10. Use according to claim 9, characterized in that they are present in the form of fish oil and/or ruminant fat.

11. Use according to claim 9 or 10, characterized in that they are present in the form of fractions of said fats and/or oils enriched by triglycerides containing branched fatty acids.

12. Use according to claims 1 to 11, characterized in that it is present in the form of an emulsion of the glyceride in aqueous phase.

13. Use according to claim 12, characterized in that it also contains 2 - 30% fractionated soy oil, safflower oil, olive oil, fish oil, ruminant fat, cottonseed oil, sunflower oil, triglyceride with an unbranched carbon chain of medium length (MCT) and/or other physiologically acceptable triglycerides of vegetable, animal or synthetic origin.

14. Use according to one of claims 12 or 13, characterized in that it also contains 0.5 - 10% acetylized mono- or diglycerides of saturated or unsaturated fatty acids with 6 to 24 carbon atoms.

**Revendications**

**Revendications pour les Etats contractants suivants : AT, BE, CH, LI, DE, DK, FR, GB, IT, LU NL, SE**

**1.** Préparation de substances nutritives pour des êtres humains, caractérisée en ce qu'elle contient au moins un glycéride difficilement soluble dans l'eau, respectivement pratiquement insoluble dans l'eau, répondant à la formule générale I

```
H2C - R1
 |
 HC - R2                I
 |
H2C - R3
```

dans laquelle un des radicaux $R_1$, $R_2$ ou $R_3$ peut représenter un radical hydroxyle ou un radical d'un acide gras saturé ou insaturé contenant de 2 à 24 atomes de carbone, et deux ou trois des radicaux $R_1$, $R_2$ ou $R_3$ représentent un radical répondant la formule générale II

```
CH3                            O
   \                           ||
    CH - (CH2)m - (CH2)n - C - O         II
   /
  R
```

dans laquelle R représente un radical méthyle ou un radical éthyle, m = 0 ou 1 et n = 0 ou un nombre entier de 2 à 18, à l'exclusion du triisobutyrate de glycéryle.

**2.** Préparation selon la revendication 1, caractérisée en ce que m = 1 et n = 0 ou un nombre entier de 2 à 6.

**3.** Préparation selon la revendication 2, caractérisée en ce que n = 0, 2 ou 4.

**4.** Préparation selon les revendications 1 à 3, caractérisée en ce que tous les radicaux $R_1$, $R_2$ et $R_3$ de la formule générale I représentent respectivement le radical de formule III

```
CH3             O
   \            ||
    CH - CH2 - C - O          III
   /
CH3
```

**5.** Préparation selon les revendications 1 à 3, caractérisée en ce que tous les radicaux $R_1$, $R_2$ et $R_3$ de la formule générale I représentent respectivement le radical de formule IV

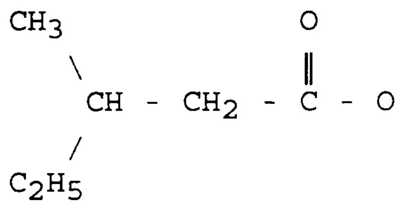

IV

**6.** Préparation selon les revendications 1 à 5, caractérisée en ce qu'elle contient le glycéride en une quantité de 0,2 - 100 % (poids/volume).

**7.** Préparation selon la revendication 6, caractérisée en ce qu'elle contient le glycéride en une quantité de 0,2 - 35 % (poids/volume).

**8.** Préparation selon les revendications 1 à 7, caractérisée en ce qu'elle contient un mélange des glycérides de formule I.

**9.** Préparation selon l'une quelconque des revendications précédentes, caractérisée en ce que les glycérides sont présents sous la forme de graisses et/ou d'huiles animales.

**10.** Préparation selon la revendication 9, caractérisée en ce qu'elle est présente sous forme d'huile de poisson et/ou de graisse de ruminant.

**11.** Préparation selon la revendication 9 ou 10, caractérisée en ce qu'elle est présente sous la forme de fractions de ces graisses et/ou de ces huiles enrichies en triglycérides contenant des acides gras ramifiés.

**12.** Préparation selon les revendications 1 à 11, caractérisée en ce qu'elle est présente sous la forme d'une émulsion du glycéride en phase aqueuse.

**13.** Préparation selon la revendication 12, caractérisée en ce qu'elle contient également de 2 à 30 % d'huile de soja fractionnée, d'huile de carthame, d'huile d'olive, d'huile de poisson, de graisse de ruminant, d'huile de semences de coton, d'huile de tournesol, de triglycérides possédant une chaîne carbonée non ramifiée de longueur moyenne (MCT) et/ou d'autres triglycérides physiologiquement acceptables d'origine végétale, animale ou synthétique.

**14.** Préparation selon l'une quelconque des revendications 12 ou 13, caractérisée en ce qu'elle contient également de 0,5 à 10% de mono- ou diglycérides acétylés d'acides gras saturés ou insaturés contenant de 6 à 24 atomes de carbone.

**15.** Utilisation d'au moins un glycéride difficilement soluble dans l'eau, respectivement pratiquement insoluble dans l'eau, répondant à la formule générale

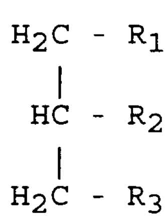

I

dans laquelle un des radicaux $R_1$, $R_2$ ou $R_3$ peut représenter un radical hydroxyle ou un radical d'un acide gras saturé ou insaturé contenant de 2 à 24 atomes de carbone, et deux ou trois des radicaux

$R_1$, $R_2$ ou $R_3$ représentent un radical répondant la formule générale II

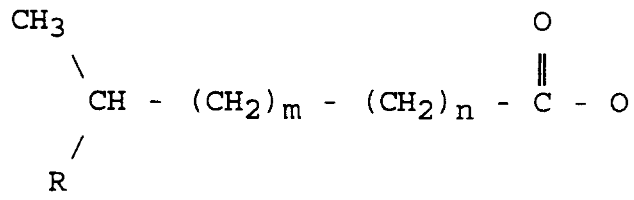

II

dans laquelle R représente un radical méthyle ou un radical éthyle, m = 0 ou 1 et n = 0 ou un nombre entier de 2 à 18, pour la formulation d'une préparation de substances nutritives pour des êtres humains qui se trouvent à l'état catabolique.

**Revendications pour les Etats contractants suivants : ES, GR**

**1.** Utilisation d'au moins un glycéride difficilement soluble dans l'eau, respectivement pratiquement insoluble dans l'eau, répondant à la formule générale I

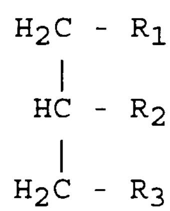

I

dans laquelle un des radicaux $R_1$, $R_2$ ou $R_3$ peut représenter un radical hydroxyle ou un radical d'un acide gras saturé ou insaturé contenant de 2 à 24 atomes de carbone, et deux ou trois des radicaux $R_1$, $R_2$ ou $R_3$ représentent un radical répondant la formule générale II

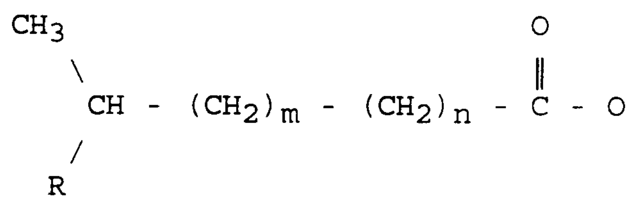

II

dans laquelle R représente un radical méthyle ou un radical éthyle, m = 0 ou 1 et n = 0 ou un nombre entier de 2 à 18, à l'exclusion du triisobutyrate de glycéryle, pour la formulation d'une préparation de substances nutritives pour des êtres humains.

**2.** Utilisation selon la revendication 1, caractérisée en ce que m = 1 et n = 0 ou un nombre entier de 2 à 6.

**3.** Utilisation selon la revendication 2, caractérisée en ce que n = 0, 2 ou 4.

**4.** Utilisation selon les revendications 1 à 3, caractérisée en ce que tous les radicaux $R_1$, $R_2$ et $R_3$ de la formule générale I représentent respectivement le radical de formule III

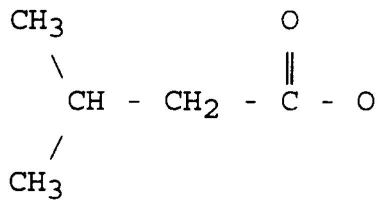

III

5. Utilisation selon les revendications 1 à 3, caractérisée en ce que tous les radicaux $R_1$, $R_2$ et $R_3$ de la formule générale I représentent respectivement le radical de formule IV

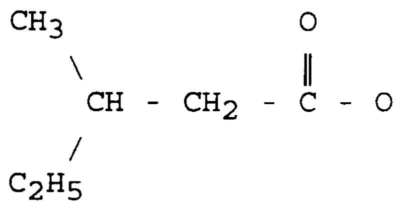

IV

6. Utilisation selon les revendications 1 à 5, caractérisée en ce que le glycéride est contenu dans la préparation en une quantité de 0,2 - 100 % (poids/volume).

7. Utilisation selon la revendication 6, caractérisée en ce que le glycéride est contenu dans la préparation en une quantité de 0,2 - 35 % (poids/volume).

8. Utilisation selon les revendications 1 à 7, caractérisée en ce qu'on utilise un mélange des glycérides de formule I.

9. Utilisation selon l'une quelconque des revendications précédentes, caractérisée en ce qu'on utilise les glycérides sous la forme de graisses et/ou d'huiles animales.

10. Utilisation selon la revendication 9, caractérisée en ce qu'on utilise les glycérides sous la forme d'huile de poisson et/ou de graisse de ruminant.

11. Utilisation selon la revendication 9 ou 10, caractérisée en ce qu'on utilise les glycérides sous la forme de fractions de ces graisses et/ou de ces huiles enrichies en triglycérides contenant des acides gras ramifiés.

12. Utilisation selon les revendications 1 à 11, caractérisée en ce qu'on formule la préparation sous la forme d'une émulsion du glycéride en phase aqueuse.

13. Utilisation selon la revendication 12, caractérisée en ce qu'on utilise également de 2 à 30 % d'huile de soja fractionnée, d'huile du carthame, d'huile d'olive, d'huile de poisson, de graisse de ruminant, d'huile de semences de coton, d'huile de tournesol, de triglycérides possédant une chaîne carbonée non ramifiée de longueur moyenne (MCT) et/ou d'autres triglycérides physiologiquement acceptables d'origine végétale, animale ou synthétique.

14. Utilisation selon l'une quelconque des revendications 12 ou 13, caractérisée en ce qu'on utilise également de 0,5 à 10% de mono- ou diglycérides acétylés d'acides gras, saturés ou insaturés contenant de 6 à 24 atomes de carbone.